Europäisches Patentamt

(19) European Patent Office

Office européen des brevets

(11) Numéro de publication: **0 217 076**

**B1**

(12) **FASCICULE DE BREVET EUROPEEN**

(45) Date de publication du fascicule du brevet: **09.01.91**

(51) Int. Cl.⁵: **C 12 P 7/06**

(21) Numéro de dépôt: **86110904.9**

(22) Date de dépôt: **07.08.86**

(54) **Production d'alcool.**

(30) Priorité: **05.09.85 CH 3831/85**

(43) Date de publication de la demande:
**08.04.87 Bulletin 87/15**

(45) Mention de la délivrance du brevet:
**09.01.91 Bulletin 91/02**

(84) Etats contractants désignés:
**AT BE DE FR GB IT LU NL SE**

(56) Documents cités:
**EP-A-0 080 095**
**EP-A-0 092 697**
**FR-A-2 465 783**
**US-A-2 356 381**

**EUROPEAN JOURNAL OF APPLIED
MICROBIOLOGY AND BIOTECHNOLOGY, vol.
16, 1982, pages 10-16, Springer-Verlag, DE; K.
TODA et al.: "Kinetics of yeast growth and
enzyme syntheses in a phosphate-limited
continuous culture"**

(73) Titulaire: **SOCIETE DES PRODUITS NESTLE S.A.
Case postale 353
CH-1800 Vevey (CH)**

(72) Inventeur: **Kalina, Vladimir
Chemin du Devin 94
CH-1012 Lausanne (CH)**

Courier Press, Leamington Spa, England.

# EP 0 217 076 B1

**Description**

La présente invention a pour objet un procédé de production d'alcool par fermentation microaérobie en continu d'un moût dans une cuve de fermentation, dans lequel on injecte du moût frais en continu dans la cuve, on soutire du moût fermenté en continu de la cuve, et l'on recycle dans la cuve la plus grande partie de la levure présente dans le moût soutiré.

On connaît divers procédés de production d'alcool par fermentation d'un milieu de culture riche en sucre fermentescible que l'on désigne par le terme de moût dans le présent exposé. Certains de ces procédés assurent une grande productivité, à savoir la production horaire d'une grande quantité d'alcool par rapport au volume de l'installation, d'autres assurent plus spécialement un bon rendement, à savoir une bonne conversion du sucre fermentescible en alcool, d'autres autorisent le travail à grande échelle et d'autres encore assurent une faible consommation d'énergie extérieure ou une construction relativement simple de l'installation.

Parmi les procédés assurant une grande productivité, on peut citer la fermentation sous pression réduite dont le désavantage est d'ordre technologique, les installations requises étant compliquées. Parmi les procédés assurant un bon rendement, on peut citer p. ex. ceux qui mettent en oeuvre plusieurs fermenteurs en cascade dans lesquels sont utilisés successivement différents types de levure, les installations requises étant là aussi compliquées et la productivité relativement modeste.

De manière générale, la fermentation en continu avec recyclage de la plus grande partie de la levure présente dans le moût soutiré se retrouve dans la plupart des procédés connus car elle devrait permettre de travailler avec une forte concentration de levure dans le moût ce qui est favorable à la productivité. Les problèmes à résoudre sont alors p. ex. l'inhibition de la fermentation si la teneur en alcool du moût est trop élevée, l'insécurité bactériologique si la teneur en alcool du moût est trop faible, la dépense en énergie de brassage si la viscosité du moût est trop grande ou si les taux de transfert d'oxygène sont trop faibles, la baisse du rendement si la levure est mal régénérée, ou la production d'une quantité de levure superflue si une quantité d'oxygène trop grande est utilisée pour maintenir le niveau d'activité de la levure dans l'installation.

Parmi les procédés connus, on peut mentionner particulièrement celui qui consiste à travailler dans au moins deux cuves de fermentation successives, les conditions de fermentation dans la première cuve étant réglées de manière à favoriser une croissance rapide de la levure, les conditions de fermentation dans la deuxième cuve étant réglées de manière à favoriser une production rapide d'alcool et la levure présente dans le moût soutiré de la deuxième cuve étant recyclée dans la première cuve. Dans ce procédé connu, une énergie extérieure considérable doit être engagée dans la première cuve pour assurer un taux de transfert d'oxygène suffisant et les conditions régnant dans la deuxième cuve favorisent l'apparition de déficiences respiratoires irréversibles de la levure auxquelles le recyclage dans la première cuve ne peut pas porter remède. Les cellules de levure présentant des déficiences respiratoires continuent à se multiplier dans la première cuve en y produisant des métabolites secondaires non souhaitées tels que le glycérol ou l'acide succinique p. ex. Pour ces raisons, ce procédé connu ne peut être réalisé qu'en utilisant des concentrations relativement faibles de levure dans le moût et l'utilisation de levure floculante n'apporte pas d'avantage particulier.

Un autre procédé connu, destiné à assurer un bon transfert d'oxygène et des conditions de fermentation favorables et homogènes tout en ne consommant qu'un minimum d'énergie extérieure, consiste à travailler en circuit fermé dans une seule cuve surmontée d'une pompe mammouth fonctionnant sous l'effet du seul gaz carbonique dégagé dans la cuve, l'oxygène étant injecté en quantité minimale au sommet d'une conduite de retour et étant complètement dissout dans le moût avant que celui-ci ne soit réinjecté dans le bas de la cuve, une pression élevée et une bonne homogénéité du moût étant assurées sur toute la hauteur de la cuve de fermentation grâce à la hauteur de la conduite de retour et à la présence d'une vanne de contre-pression au sommet de la cuve de fermentation, la levure utilisée étant floculante, la séparation de la levure présente dans le moût prélevé s'effectuant à l'extérieur de la cuve de fermentation dans un récipient de décantation sous pression, et la pression dans le récipient de décantation de même que le recyclage de la levure étant assurés également par la hauteur de la conduite de retour. Ce procédé connu se laisse réaliser à grande échelle tout en assurant une bonne productivité et un bon rendement. Cependant, un enrichissement du moût en cellules de levure présentant une dégénérescence du système respiratoire voire une déficience respiratoire irréversible est inévitable à la longue ce qui limite la durée praticable d'un tel processus de fermentation à environ 1 à 2 semaines au-delà desquelles le rendement et la productivité sont diminués notamment par la production de métabolites secondaires tels que le glycérol ou l'acide succinique p. ex.

La présente invention a pour but de proposer un procédé de production d'alcool par fermentation en continu permettant d'assurer un rendement particulièrement bon tout en présentant une grande stabilité à long terme. Elle a en particulier pour but de proposer un procédé qui soit réalisable durant un temps considérablement plus long que 1 à 2 semaines sans que la productivité et le rendement diminuent.

A cet effet, le procédé selon la présente invention est caractérisé par le fait que l'on inhibe la croissance de la levure dans la cuve en limitant la concentration de phosphate assimilable dans le moût et l'on régénère la levure dans la cuve en injectant de la levure fraîche en continu dans la cuve.

On a constaté qu'une limitation de la concentration du phosphate dans le moût permet de manière

2

EP 0 217 076 B1

surprenante de limiter la croissance de la levure utilisée pour la production d'alcool sans provoquer la production de métabolites secondaires. Ceci permet d'obtenir un rendement, autrement dit un facteur de conversion en alcool du sucre fermentescible contenu dans le moût proche du maximum théorique qui est d'environ 51% en poids. Le présent procédé permet d'obtenir en particulier un rendement d'environ 49% ou plus qui est supérieur aux rendements obtenus avec la plupart des procédés connus mentionnés ci-dessus qui sont généralement compris entre environ 44 et 48%. Et l'on a constaté qu'en régénérant simultanément la levure dans la cuve en y injectant en continu de la levure fraîche en quantités minimes, notamment en injectant par heure un poids de levure fraîche correspondant à une fraction de % du poids de levure contenu dans la cuve, on peut maintenir le rendement et la productivité au même niveau pratiquement indéfiniment ou en tous les cas durant un temps considérablement plus long que les 1 ou 2 semaines caractéristiques de la durée maximum possible de la plupart des procédés connus mentionnés ci-dessus.

La présente combinaison des deux caractéristiques essentielles du présent procédé, à savoir l'inhibition de la croissance des levures par la limitation du phosphate assimilable dans le moût et la régénération de la levure par l'addition en continu de levure fraîche permet donc de manière surprenante d'assurer à la fois un rendement particulièrement bon et une grande stabilité à long terme. Un autre avantage du présent procédé est qu'il permet d'obtenir ces résultats surprenants aussi bien avec des levures floculantes qu'avec des levures non floculantes. Encore un autre avantage de ce procédé est que, dans une forme d'exécution préférée, il peut être réalisé en n'utilisant que la seule quantité d'oxygène nécessaire au maintien de l'activité de la levure. On peut donc diminuer la quantité d'oxygène utilisée, même par rapport à celle utilisée dans le dernier des procédés connus ci-dessus, sans risquer la production de métabolites secondaires indésirés tels que le glycérol ou l'acide succinique p. ex.

Pour mettre en oeuvre le présent procédé, on peut utiliser comme matière première un moût présentant de préférence une teneur en sucre fermentescible comprise entre 100 et 200 g/l outre les substances nutritives nécessaires à la levure. Si la teneur en sucre fermentescible du moût est inférieure à 100 g/l, la faible teneur en alcool du moût soutiré rend inutilement chère l'étape ultérieure de séparation de l'alcool du moût, par distillation p. ex. Si la teneur en sucre fermentescible du moût est supérieure à 200 g/l, la forte teneur en alcool du moût dans la cuve ralentit exagérément la fermentation et diminue la productivité.

On injecte de préférence par heure dans la cuve de fermentation un volume de moût frais correspondant à 0,1-0,3 fois le volume du moût présent dans la cuve. Si l'on injecte par heure moins de moût frais que la plus petite quantité ainsi définie, on peut peut-être travailler avec une plus forte concentration d'alcool dans la cuve, mais à un rythme trop lent qui diminue la productivité. Si l'on injecte par heure plus de moût frais que la plus grande quantité ainsi définie, on ne peut pas travailler avec une concentration d'alcool suffisante dans la cuve ce qui renchérit inutilement l'étape ultérieure de séparation de l'alcool du moût.

En ce qui concerne la levure utilisée, on peut la choisir parmi les levures connues pour leurs bonnes aptitudes à la production d'alcool. Selon le type de fermenteur utilisé, on peut choisir une levure connue pour ses bonnes aptitudes à la formation d'agrégats qui facilitent leur sédimentation, autrement dit une levure floculante, ou une levure qui n'a pas particulièrement tendance à former des agrégats, autrement dit une levure non floculante. Lorsqu'on utilise une levure floculante, on peut la séparer avantageusement du moût par décantation, notamment par une décantation sous pression qui comprime le gaz carbonique présent dans le moût et l'empêche de faire flotter les agrégats. Lorsqu'on utilise une levure non floculante, on peut la séparer du moût par centrifugation ou filtration p. ex.

Le moût dans la cuve peut contenir 30-80 g de poids sec de levure par litre. Si l'on travaille avec moins de 30 g/l, on diminue inutilement la productivité. Si l'on travaille avec plus de 80 g/l, on s'expose à des problèmes de viscosité dans la cuve et lors de l'opération de séparation de la levure du moût soutiré.

Pour régénérer la levure dans la cuve, autrement dit pour maintenir l'activité de la levure dans le cadre du présent procédé, on injecte de préférence par heure dans la cuve un poids de levure fraîche correspondant à environ 0,2-0,5% du poids de la levure contenue dans la cuve. Ces chiffres correspondent à un renouvellement complet de la levure dans la cuve en l'espace d'environ 8-20 j.

Pour inhiber la croissance de la levure dans la cuve, on limite donc la concentration de phosphate assimilable dans le moût. De préférence, on limite la concentration de phosphate assimilable à une valeur comprise entre 0,01 et 0,2 g/l dans le moût frais injecté. Avec moins de 0,01 g/l, on risque de ne pas assurer le maintien de l'activité désirée de la levure. Avec plus de 0,2 g/l, on risque de ne pas obtenir l'effet d'inhibition de la croissance souhaitée.

Dans une forme d'exécution préférée du présent procédé, on limite la concentration de phosphate assimilable en ajoutant au moût frais injecté ou en injectant directement dans la cuve un sel soluble d'aluminium, tel que du sulfate ou du chlorure d'aluminium p. ex., en concentration molaire au moins 3 fois supérieure à celle du phosphate présent dans ledit moût frais. De la sorte, la plus grande partie du phosphate est précipitée sous forme de sel d'aluminium et la concentration de phosphate en solution dans le moût ne peut pas dépasser une valeur très faible qui est fonction de la constante d'équilibre du système. Et même si les faibles quantités de phosphate en solution se reforment au fur et à mesure de leur consommation par la levure, celle-ci doit fournir continuellement un gros effort pour trouver l'accès à cette faible quantité et l'effet d'inhibition de la croissance est ainsi assuré.

3

Le présent procédé peut être mis en oeuvre avec pratiquement toutes les installations de fermentation connues conçues pour travailler en continu.

Il se prête notamment très bien à la mise en oeuvre à l'aide d'un appareil à une cuve sous pression surmontée d'une pompe mammouth dans lequel le moût circule continuellement en circuit fermé sous l'effet du seul gaz carbonique dégagé par la fermentation.

Les exemples ci-après sont présentés à titre d'illustration ou de comparaison.

Exemple 1

On utilise un fermenteur comportant une cuve de fermentation de 15 litres présentant une hauteur de 50 cm et un diamètre de 20 cm, des dispositifs d'injection de moût frais, de levure fraîche et de gaz dans la cuve, un dispositif de soutirage du moût fermenté et un dispositif d'évacuation de gaz de la cuve, un récipient de décantation extérieur à la cuve et un dispositif de recyclage de la levure capable de maintenir à un niveau constant le poids de levure contenu dans la cuve.

On maintient à 10 l le volume de moût contenu dans la cuve de fermentation. On injecte dans la cuve 1,35 l/h de moût frais présentant la composition suivante, en g/l :

| saccharose | 165 | $CaCl_2.2H_2O$ | 0,05 |
| extrait de levure | 1 | $MnSO_4.7H_2O$ | 0,01 |
| $K_2SO_4$ | 0,5 | $FeSO_4.7H_2O$ | 0,01 |
| $MgSO_4.7H_2O$ | 0,5 | $ZnSO_4.7H_2O$ | 0,005 |
| $(NH_4)_2SO_4$ | 0,5 | $H_3PO_4$ | 0,05 |

On injecte également dans la cuve 150 ml/h d'un moût contenant 3 g de poids sec de levure fraîche. On obtient cette levure fraîche en cultivant la levure Saccharomyces cerevisiae CBS 2961 en conditions aérobies avec un rendement de 50% sur saccharose. En d'autres termes, on uilise 6 g de saccharose pour produire ces 3 g de poids sec de levure fraîche.

On injecte enfin dans la cuve 2,4 l/h d'air en mélange avec de l'azote de manière à obtenir un total de 180 litres de gaz évacués par h du fermenteur. On agite le moût dans la cuve à l'aide de deux turbines à quatre ailettes carrées de 90 mm de côté tournant à 300 tours/min. On maintient le pH du moût dans la cuve à une valeur constante de 4,7 par addition d'acide sulfurique ou d'ammoniaque. On maintient la température du moût dans la cuve à 33°C.

On soutire de la cuve 4 l/h de moût fermenté contenant 65 g/l de poids sec de levure. On conduit le moût soutiré à un récipient de décantation de 5 l dans lequel règne une pression de 3 bar. On recycle dans la cuve 2,5 l/h de suspension concentrée de levure tout en maintenant constant le poids de levure contenu dans la cuve. On recueille 1,5 l/h de moût contenant par litre 70 g d'alcool; moins de 1 g de saccharose et moins de 0,5 g de matière sèche de levure.

La quantité d'alcool récupérable entraînée avec les gaz sortant du fermenteur étant de 6,3 g/h, on obtient ainsi un rendement de 49% et une productivité de 11 g d'alcool par heure et par litre de volume effectif de la cuve. Le rapport entre le poids sec de levure fraîche injectée et le poids d'alcool produit est inférieur à 0,03.

Après un mois de fonctionnement ininterrompu du fermenteur dans les mêmes conditions, on n'observe aucune baisse ni du rendement ni de la productivité.

Exemple 2

On procède de la manière décrite à l'exemple 1, à l'exception du fait que le moût frais injecté contient 0,2 g/l de $H_3PO_4$ au lieu de 0,05 g/l et que l'on injecte en outre directement dans la cuve 2,7 g/h de $Al_2(SO_4)_3.18H_2O$, ce qui correspond à une concentration molaire d'Al 3 fois supérieure à celle d'$H_3PO_4$. On obtient le même rendement et la même productivité qu'à l'exemple 1.

Exemple comparatif

On procède de manière semblable à celle décrite à l'exemple 1, à l'exception du fait que l'on injecte dans la cuve 2,25 l/h de moût frais contenant par litre 130 g de saccharose au lieu de 165 g et 0,8 g de $H_3PO_4$ au lieu de 0,05 g, on n'injecte pas de levure fraîche et l'on injecte de l'air à raison de 90 ml/h au lieu de 40 ml/h.

On soutire de la cuve 4,75 l/h de moût fermenté contenant 50 g/l de poids sec de levure. On réalise la décantation et l'on recycle également 2,5 l/h de suspension concentrée de levure mais on évacue du récipient de décantation 13,8 g/h de poids sec de levure. On recueille 2,25 l/h de moût contenant par litre 55 g d'alcool et également moins de 1 g de saccharose et moins de 0,5 g de matière sèche de levure.

La quantité d'alcool récupérable entraîné avec les gaz sortant du fermenteur étant de 5 g/h, on obtient ainsi un rendement de 44% et une productivité de 13 g d'alcool par heure et par litre de volume effectif de la cuve. Le rapport entre le poids sec de levure évacué et le poids d'alcool produit est de 0,11.

Si l'on désire obtenir une plus forte teneur en alcool du moût fermenté, on doit augmenter la teneur en sucre fermentescible du moût frais injecté et la teneur en levure du moût dans la cuve. Ceci conduit rapidement à des problèmes d'instabilité car l'on doit simultanément augmenter considérablement l'aération du moût pour éviter une déficience respiratoire de la levure qui provoquerait une baisse du rendement et de la productivité.

N.B.: Dans le présent exposé, on entend par l'expression " levure fraîche" une levure qui a été obtenue par fermentation aérobie et qui possède un système respiratoire intact, autrement dit une activité respiratoire élevée.

**Revendications**

1. Procédé de production d'alcool par fermentation microaérobie en continu d'un moût dans une cuve de fermentation, dans lequel on injecte du moût frais en continu dans la cuve, on soutire du moût fermenté en continu de la cuve, et l'on recycle dans la cuve la plus grande partie de la levure présente dans le moût soutiré, caractérisé par le fait que l'on inhibe la croissance de la levure dans la cuve en limitant la concentration de phosphate assimilable à une valeur comprise entre 0,01 et 0,2 g/l dans le moût frais injecté et l'on régénère la levure dans la cuve en injectant de la levure fraîche en continu dans la cuve, le moût dans la cuve contenant 30-80 g de poids sec de levure par litre.

2. Procédé selon la revendication 1, caractérisé par le fait que l'on injecte par heure dans la cuve un volume de moût frais correspondant à 0,1-0,3 fois le volume de moût présent dans la cuve, la teneur en sucre fermentescible du moût frais étant de 100-200 g/l.

3. Procédé selon la revendication 1, caractérisé par le fait que l'on injecte par heure dans la cuve un poids de levure fraîche correspondant à 0,2-0,5 % du poids de la levure contenue dans la cuve.

4. Procédé selon la revendication 1, caractérisé par le fait que l'on limite la concentration de phosphate assimilable en ajoutant au moût frais injecté un sel soluble d'aluminium en concentration molaire au moins 3 fois supérieure à celle du phosphate présent dans ledit moût frais.

5. Procédé selon la revendication 1, caractérisé par le fait que la levure utilisée est une levure floculante que l'on sépare du moût par décantation.

6. Procédé selon la revendication 1, caractérisé par le fait que la levure utilisée est une levure non floculante que l'on sépare du moût par centrifugation.

**Patentansprüche**

1. Verfahren zur Herstellung von Alkohol durch kontinuierliches Vergären, unter mikroaeroben Bedingungen, einer Maische in einem Gärgefäß, bei welchem Verfahren man frische Maische kontinuierlich in das Gärgefäß einführt, vergorene Maische kontinuierlich aus dem Gärgefäß abzieht und den größten Teil der in der abgezogenen Maische vorhandenen Hefe im Kreislauf in das Gärgefäß rückführt, dadurch gekennzeichnet, daß man das Wachstum der Hefe in dem Gärgefäß hemmt, indem man die Konzentration von assimilierbarem Phosphat in der eingeführten frischen Maische auf einen Wert zwischen 0,01 und 0,2 g/l beschränkt, und daß man die Hefe in dem Gägefäß regeneriert, indem man kontinuierlich frische Hefe in das Gärgefäß einbringt, wobei die Maische in dem Gärgefäß einen Hefegehalt von 30 bis 80 g/l, bezogen auf die Trockensubstanz, aufweist.

2. Verfahren nach Anspruch 1, dadurch gekennzeichnet, daß man pro Stunde in das Gärgefäß ein Volumen von frischer Maische einführt, welches dem 0,1- bis 0,3-fachen des in dem Gärgefäß vorhandenen Maischevolumens entspricht, wobei der Gehalt der frischen Maische an vergärbarem Zucker 100 bis 200 g/l beträgt.

3. Verfahren nach Anspruch 1, dadurch gekennzeichnet, daß man pro Stunde in das Gärgefäß einen Gewichtsanteil von freischer Hefe einbringt, welcher 0,2- bis 0,5 Gew.-% der in dem Gärgefäß enthaltenen Hefe entspricht.

4. Verfahren nach Anspruch 1, dadurch gekennzeichnet, daß man die Konzentration an assimilierbarem Phosphat dadurch beschränkt, daß man zu der eingeführten frischen Maische in lösliches Aluminiumsalz von einer molaren Konzentration hinzufüht, welche wenigstens dreimal so groß wie jene des in der frischen Maische vorliegenden Phosphates ist.

5. Verfahren nach Anspruch 1, dadurch gekennzeichnet, daß die verwendete Hefe eine Flockhefe ist, die man durch Dekantieren von der Maische abtrennt.

6. Verfahren nach Anspruch 1, dadurch gekennzeichnet, daß die verwendete Hefe eine nicht zur Flockenbildung neigende Hefe ist, welche man durch Zentrifugieren von dere Maische abtrennt.

**Claims**

1. A process for the production of alcohol by continuous microaerobic fermentation of a must in a fermentation vat, in which fresh must is continuously injected into the vat, fermented must is continuously run off from the vat and most of the yeast present in the must run off is recycled into the vat, characterized in that the growth of the yeast in the vat is inihibited by limiting the concentration of assimilable phosphate

5

in the fresh must injected to a value of 0.01 to 0.2 g/l and the yeast in the vat is regenerated by continuous injection of fresh yeast into the vat, the must in the vat containing 30-80 g dry weight of yeast per litre.

2. A process as claimed in claim 1, characterized in that a volume of fresh must corresponding to 0.1-0.3 times the volume of must present in the fermentation vat is injected hourly into the vat, the fermentable sugar content of the must being 100-200 g/l.

3. A process as claimed in claim 1, characterized in that a weight of fresh yeast corresponding to 0.2-0.5% of the weight of the yeast in the vat is injected hourly into the vat.

4. A process as claimed in claim 1, characterized in that the concentration of assimilable phosphate is limited by addition to the fresh must injected of a soluble aluminium salt in a molar concentration at least three times higher than that of the phosphate present in said fresh must.

5. A process as claimed in claim 1, characterized in that the yeast used is a flocculating yeast which is separated from the must by decantation.

6. A process as claimed in claim 1, characterized in that the yeast used is a non-flocculating yeast which is separated from the must be centrifuging.